# FASCICULE DE BREVET EUROPEEN

(11) **EP 0 516 494 B1**
(45) Date de publication et mention de la délivrance du brevet: **09.08.1995**
(21) Numéro de dépôt: 92401104.2
(22) Date de dépôt: 17.04.1992
(51) Int. Cl.: A61B 17/39

(54) **Instrument chirurgical à pince miniature déportée notamment pour coeliochirurgie**
Miniaturisierte Zange als chirurgisches Instrument für Coeliochirurgie
Miniaturised forceps medical instrument for celio-surgery

(30) Priorité: 31.05.1991 FR 9106569
(43) Date de publication de la demande: 02.12.1992
(73) Titulaire: MIKROLAND Société Anonyme, F-74100 Ville-Lagrand (FR); WALTON MEDICAL S.A.R.L., F-52000 Chaumont (FR)
(72) Inventeur: Gladkoff, Hervé, F-74250 Viuz-en-Sallaz (FR)
(74) Mandataire: Martinet & Lapoux

(56) Documents cités:
- EP-A- 0 210 125
- FR-A- 598 149
- FR-A- 2 526 303
- GB-A- 2 161 082
- GB-A- 2 194 748

## Description

La présente invention concerne un instrument chirurgical du type pince comprenant des mors miniatures déportés par rapport à deux branches commandant à distance la fermeture et l'ouverture des mors de pince à travers un tube de liaison.

Un tel instrument est utilisé en dépendance de la forme des mors par exemple comme forceps pour saisir ou rétracter un organe, comme dissecteur pour retirer des tissus, ou bien encore comme des ciseaux pour couper des vaisseaux et artères. En particulier, l'instrument est utilisé en coeliochirurgie notamment pour choleocystostomie au cours de laquelle il est parfois nécessaire de cautériser ou disséquer des vaisseaux au moyen d'un courant électrique.

L'instrument est composé essentiellement d'une branche fixe à laquelle est fixée une extrémité du tube de liaison dont l'autre extrémité supporte les mors, d'une branche mobile articulée à la branche fixe et solidaire d'une tige glissant dans le tube de liaison pour déplacer au moins l'un mobile des mors, d'une crémaillère sur l'une des branches coopérant avec un ergot sur l'autre branche pour arrêter la branche mobile par rapport à la branche fixe et donc arrêter le mors mobile à une position souhaitée, et de moyens de liaison électrique fixés à la branche fixe pour conduire un courant vers les mors.

Du point de vue ergonomique, deux types de position relative des branches par rapport au tube sont déjà connues.

Selon un premier type d'instrument, la branche fixe forme avec le tube de liaison un angle obtu faible, sensiblement supérieur à un angle droit, et la branche mobile est située à l'extérieur de cet angle obtu. L'anneau de branche mobile est alors saisi par le pouce de la main qui exerce lui-même le déplacement de la branche mobile par rapport à la branche fixe. Le mouvement imposé du pouce est ainsi contraire à la prise naturelle de tout objet par la main au cours de laquelle les autres doigts de la main se rapprochent du pouce sensiblement immobile.

Selon un second type d'instrument, la branche fixe et le tube de liaison délimitent un angle obtu largement ouvert, et la branche mobile peut osciller à l'intérieur de cet angle obtu ouvert. Le pouce passant dans l'anneau de la branche fixe demeure immobile lorsque la branche mobile est déplacée par d'autres doigts de la main.

L'invention a trait plus particulièrement à ce second type d'instrument.

Dans ce second type d'instrument, les branches sont métalliques, par exemple en acier inoxydable, et sont revêtues d'une couche isolante électrique afin d'isoler le chirurgien du courant traversant notamment la branche fixe. La couche isolante est par exemple obtenue par immersion totale des branches dans un bain de polyamide, tel que RILSAN (marque déposée). L'isolation externe du tube de liaison propre à pénétrer dans le corps du patient peut être également obtenue au moyen d'une gaine en RILSAN.

A l'usage, la couche isolante sur les branches particulièrement au niveau des anneaux de préhension des branches ne résiste pas aux nombreuses manipulations et nettoyages antiseptiques et tend à disparaître. L'isolation électrique n'est donc plus assurée. Il en est de même pour la pellicule isolante recouvrant le tube de liaison qui ne garantit plus une conduction électrique optimale jusqu'aux mors.

Le problème de l'isolation électrique est plus accentué au niveau de la crémaillère qui est également métallique. En effet, la crémaillère comporte des crans qui coopèrent avec un ergot, ou une autre crémaillère assujettie à la branche mobile, saillant de la branche mobile. L'ergot est susceptible de riper sur les crans de la crémaillère si bien que la couche isolante ne peut être pratiquement jamais maintenue sur les crans. Comme la crémaillère est fléchie par l'un des doigts de la main pour dégager l'ergot de la branche mobile afin de déplacer celle-ci, un doigt de la main est susceptible de venir au contact des crans non isolés.

Un autre inconvénient inhérent à la composition en acier des branches est le poids excessif de l'instrument qui implique une tenue ferme et constante de l'instrument par le praticien pour éviter tout traumatisme interne.

Dans ce type d'instrument chirurgical connu, la crémaillère dont une extrémité est fixée à la branche fixe, croise complètement et latéralement la branche mobile afin qu'une extrémité de la crémaillère devant la branche mobile puisse être saisie pour dégager l'ergot de branche mobile par flexion de la crémaillère. Le doigt agissant sur la crémaillère est au contact des crans et peut être blessé non seulement par les crans mais également par coincement entre la crémaillère et le corps de la branche mobile.

S'agissant de l'ergonomie, outre le poids de l'instrument et les risques de blessure par la crémaillère, le profil de la branche mobile est inconfortable pour les phalanges des doigts à côté de l'anneau de préhension. L'angle entre le corps de branche mobile et la périphérie de l'anneau est aigu. La tenue préhensive de l'instrument est faible et impose une crispation de la main. En outre, l'angle obtu formé entre la branche fixe et le tube de liaison est inférieur sensiblement à 100°, ce qui fatigue la main du chirurgien.

Les moyens de liaison électrique comportent un embout métallique de prise électrique, mâle ou femelle, rapporté sur le nez de la branche fixe recevant le tube de liaison. Le câble électrique connectable à cet embout est susceptible de tomber sur la main du chirurgien ce qui l'entrave au cours du geste opératoire. En outre, un tel embout saillant sur l'instrument pose à nouveau le problème d'isolation électrique.

La présente invention vise à fournir un instrument chirurgical n'offrant pas les inconvénients évoqués ci-dessus et particulièrement à résoudre simultanément les problèmes d'isolation électrique, de poids d'instrument et d'accessibilité de crémaillère.

A cette fin, un instrument chirurgical comprenant :
- une première branche ayant un coude le partageant en un nez et un corps dont l'extrémité opposée au coude comporte un premier anneau de préhension,
- une seconde branche articulée autour d'un pivot fixé sensiblement à l'intérieur du coude de la première branche, la seconde branche comportant de part et d'autre du pivot, une tête et un corps dont l'extrémité opposée au pivot comporte un second anneau de préhension,
- un tube de liaison ayant une première extrémité supportant deux mors dont l'un au moins est mobile, et une seconde extrémité fixée au nez,
- une tige traversant le tube de liaison et reliant le mors mobile à la tête de seconde branche,
- une crémaillère ayant une première extrémité coopérant avec un moyen d'arrêt relié au corps de seconde branche, et une seconde extrémité fixée au corps de première branche, et
- des moyens de liaison électrique solidaires de la première branche pour relier électriquement les mors à des moyens d'alimentation électrique monopolaire,
est caractérisé en ce que les première et seconde branches sont en matière isolante électriquement pour isoler la crémaillère et le moyen d'arrêt par rapport aux moyens de liaison électrique, et la crémaillère et le moyen d'arrêt sont logés à l'intérieur des corps de branche.

Par nature, la matière isolante des branches, telle que matière plastique, confère un poids faible à l'instrument, de l'ordre de 40 grammes, et résoud complètement le problème d'isolation électrique. En effet, la crémaillère et le moyen d'arrêt bien qu'ils puissent être en matériau conducteur résistant, tel qu'acier inoxydable, ne sont pas en contact direct avec le tube de liaison ou des éléments des moyens de liaison électrique.

Aucune blessure n'est conférée par la crémaillère puisque celle-ci est complètement contenue dans les branches, quelle que soit la position de la seconde branche par rapport à la première branche.

De préférence, la première branche est constituée de deux demi-coques sensiblement plates et moulées, et la seconde branche est monolithique et moulée. Le moulage par exemple par injection plastique permet de réduire le coût de l'instrument comparativement à un usinage. La composition de la première branche en deux demi-coques se recouvrant autorise à insérer des éléments métalliques notamment pour les moyens de liaison électrique qui sont complètement enfermés dans la première branche après assemblage des demi-coques.

Selon une réalisation préférée, les première et seconde branches comportent les caractéristiques suivantes afin que la crémaillère soit inaccessible tout en permettant une oscillation limitée de la branche mobile :
- la première branche comporte un logement ouvert ménagé à l'intérieur du coude et du corps de première branche pour loger la tête et une partie du corps de seconde branche, et ladite partie du corps de seconde branche comportant une mortaise recevant la seconde extrémité de la crémaillère ;
- l'amplitude de l'oscillation de la seconde branche autour du pivot est limitée par une première portion de fond du logement dans le corps de première branche faisant office de butée pour un côté du corps de seconde branche, et par une seconde portion de fond du logement faisant office de butée pour un côté de la tête de seconde branche, ladite amplitude étant de préférence de 20° environ.

Le moyen d'arrêt selon l'invention n'est pas débrayé de la crémaillère suite à une flexion de celle-ci, et peut être dégagé complètement de la crémaillère afin de ne pas raboter les crans de la crémaillère et donc d'augmenter la longévité de celle-ci. Selon une réalisation préférée, le moyen d'arrêt est une gâchette pivotante autour d'un second pivot fixé dans le corps de la seconde branche. Ladite gâchette est partagée par le second pivot en un premier bras logé dans une première mortaise ménagée dans le corps de seconde branche et recevant la seconde extrémité de la crémaillère, et un second bras logé dans une seconde mortaise ménagée dans le second anneau. Le premier bras est engagé avec des crans de la seconde extrémité de crémaillère et le second bras est partiellement saillant à l'intérieur du second anneau sous l'action d'un moyen de ressort. D'autre part, le premier bras est dégagé des crans de crémaillère et le second bras est contenu complètement dans la seconde mortaise sous l'action d'une poussée exercée de l'intérieur du second anneau sur le second bras et à l'encontre du moyen de ressort.

L'ergonomie de l'instrument selon l'invention est de préférence particulièrement soignée tant au niveau de la gâchette qu'au niveau du contour de la seconde branche mobile :
- le second bras de gâchette a un profil épousant sensiblement celui de l'anneau ;
- la seconde mortaise est située dans un côté du second anneau orientée vers les mors et à l'opposé du premier anneau ;
- la seconde branche présente un chant sensiblement sinusoïdal orienté vers les mors et à l'opposé du premier anneau, ledit chant comportant une portion convexe centrale formée par un côté du second anneau, une première portion concave située devant la première extrémité de crémaillère, et une seconde portion concave formée par un côté d'une anse liée au second anneau ;
- la portion convexe centrale du chant sinusoïdal borde la seconde mortaise.

Les index et annulaire de la main sont ainsi appliqués confortablement dans les première et seconde portions concaves de chant sinusoïdal et peuvent exercer des forces antagonistes à celle exercée sur le second bras de la gâchette par le majeur de la main à l'intérieur du second anneau.

Selon une réalisation préférée, les moyens de liaison électrique comportent un premier élément conducteur logé dans l'arrière du corps de première branche au niveau du premier anneau pour être assemblé avec un second élément conducteur de prise de raccordement électrique monopolaire relié aux moyens d'alimentation, et un fil électrique cheminant dans un canal qui s'étend longitudinalement dans la première branche et isole le fil de la crémaillère, ledit fil ayant une première extrémité liée au premier élément conducteur et une seconde extrémité située dans le nez et liée au tube de liaison. Les premier et second éléments conducteurs étant à l'arrière de la branche fixe, le câble de raccordement électrique à l'instrument sort ainsi sous la main du chirurgien sans le gêner. Le premier élément conducteur et le fil électrique sont complètement "noyés" dans la première branche, par exemple dans l'une des demi-coques ne contenant pas la crémaillère, ce qui assure l'isolation électrique avec l'extérieur.

L'étanchéité de l'intérieur du tube de liaison et donc du corps du patient par rapport à l'environnement extérieur consiste, de préférence, en ce que la seconde extrémité de tube est emmanchée dans un fourreau logé dans le nez de première branche et est prolongée dans le fourreau par un joint traversé à frottement par la tige.

D'autres avantages de la présente invention apparaîtront plus clairement à la lecture de la description suivante de plusieurs réalisations préférées de l'invention en référence aux dessins annexés correspondants dans lesquels :
- la Fig. 1 est une vue de face partiellement en coupe suivant un plan horizontal de quasi-symétrie d'un instrument chirurgical selon l'invention, la demi-coque sus-jacente de la première branche étant omise, et la seconde branche étant représentée complètement en coupe ;
- les Figs. 2A et 2B sont des vues de face schématiques de l'instrument montrant la seconde branche mobile et un mors mobile respectivement en position de pince fermée et position de pince ouverte ;
- les Figs. 3A et 3B sont des vues partiellement en coupe analogues à la Fig. 1 montrant l'instrument en position de pince fermée respectivement lorsque le moyen d'arrêt, dit gâchette, est engagé dans la crémaillère et lorsque le moyen d'arrêt est dégagé de la crémaillère ; et
- les Figs. 4A et 4B sont des vues partiellement en coupe analogues à la Fig. 1 montrant l'instrument en position de pince ouverte respectivement lorsque le moyen d'arrêt est dégagé de la crémaillère et lorsque le moyen d'arrêt est engagé dans la crémaillère.

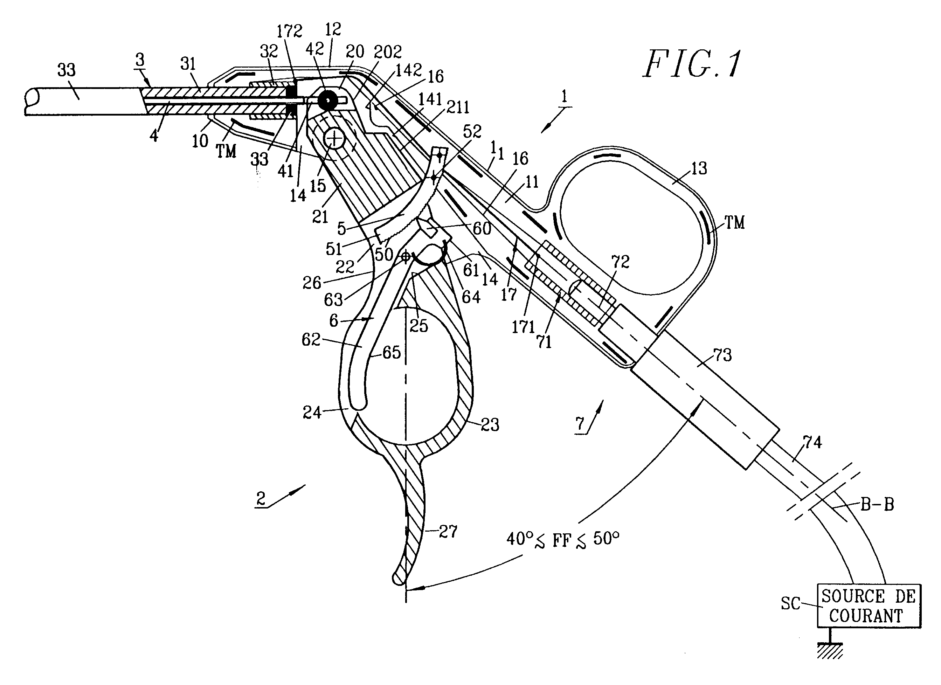

En référence à la Fig.1, un instrument de chirurgie du type pince de coeliochirurgie comprend essentiellement une première branche appelée branche fixe 1, une seconde branche, appelée branche mobile 2 qui est articulée à la poignée fixe, un tube de liaison 3 reliant la branche fixe à un mors fixe MF, une tige 4 montée à coulissement libre dans le tube et reliant la branche mobile à un mors mobile MM articulé au mors fixe, et une crémaillère 5 coopérant avec un moyen d'arrêt de la poignée mobile sous la forme d'une gâchette 6 pour bloquer la branche mobile à une position souhaitée par rapport à la branche fixe.

La branche fixe 1 est constituée de deux demi-coques 1₁ et 1₂ sensiblement plates, qui symétriques en épaisseur et contour par rapport à un plan horizontal parallèle à la Fig.1. Dans cette figure, l'une sous-jacente 1₁ des demi-coques a été représentée en détail avec ses divers évidements recouverts par l'autre sus-jacente demi-coque 1₂. Les demi-coques sont assemblées face-à-face par des tenons et mortaises TM prévus à la périphérie des deux demi-coques, ou par des bossages minces reliés par soudure ultrason.

Les demi-coques de branche fixe 1₁ et 1₂ et la branche mobile 2 qui est monolithique, sont en matière isolante électriquement qui peut résister à un nettoyage répété sous autoclave à 135° pendant 10 minutes au moins, sans aucune détèrioration. Typiquement, ces branches sont en matière plastique injectée à très haute performance mécanique.

La branche fixe 1 présente un nez 10 s'étendant suivant l'axe du tube de liaison 3, ici horizontal, un corps longilique 11 s'étendant le long d'un axe BB et formant un angle obtu AO avec le tube 3 et le nez 10 au niveau d'un coude 12 à l'arrière du nez, et un anneau oblong de préhension 13 situé sur l'arrière du corps, à l'extrémité opposée au coude et à l'extérieur de l'angle AO.

Dans la branche fixe 1 est prévue un logement 14 pour recevoir sensiblement la moitié de la branche mobile 2 contenant la tête 20 et l'avant du corps 21 de cette branche mobile. Le logement 14 est formé par deux évidements superficiels en regard, menagés dans les demi-coques 1₁ et 1₂, comme tous les autres conduits et logements internes à la branche fixe et cités dans la suite de la description. Le logement 14 forme une cavité ouverte latéralement vers l'intérieur de l'angle obtu AO et s'étend à partir de l'arrière du nez 10 et du coude 12 jusqu'en deça de l'anneau 13. Sensiblement à la verticale du coude 12 et perpendiculairement au plan de la branche 1 est fixé un pivot d'articulation 15 qui traverse le logement 14. La branche mobile 2 est montée à rotation autour de ce pivot 15.

La branche mobile 2 comporte une tête 20 en forme de chape dont la queue est montée tournante autour du pivot 15, un corps 21 prolongeant la queue de chape et coudé vers l'intérieur de l'angle AO en s'éloignant de l'arrière du corps de branche mobile 11, et un anneau oblong de préhension 23 prolongeant l'arrière du corps 21. La tête 20 est logée dans le logement de branche fixe 14, au niveau du coude 12, afin que la chape de tête soit traversée par l'extrémité arrière de la tige 4. Sensiblement une moitié du corps de branche mobile 21 peut pénétrer dans la portion du logement 14 ménagée dans le corps 11.

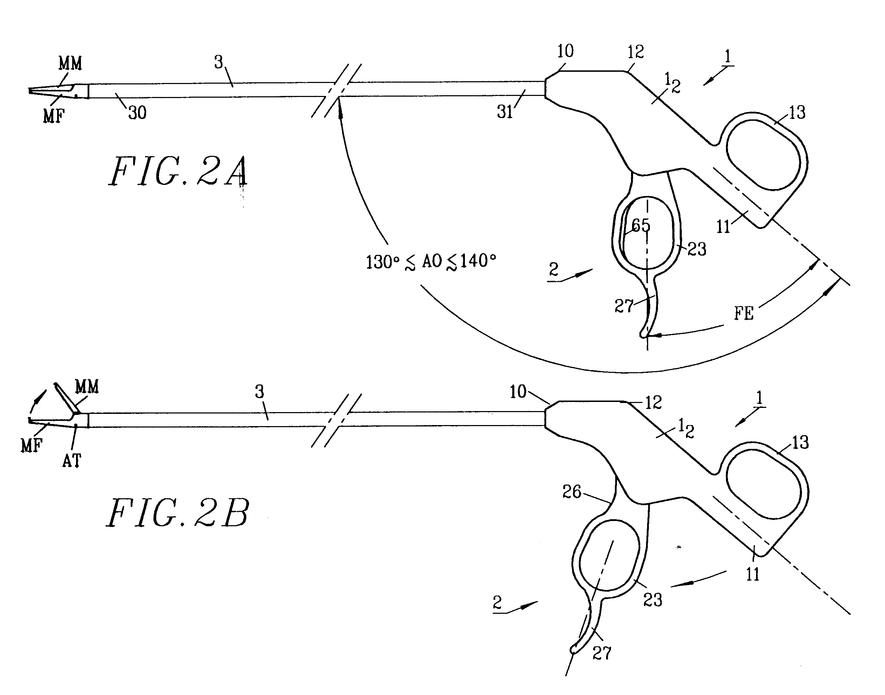

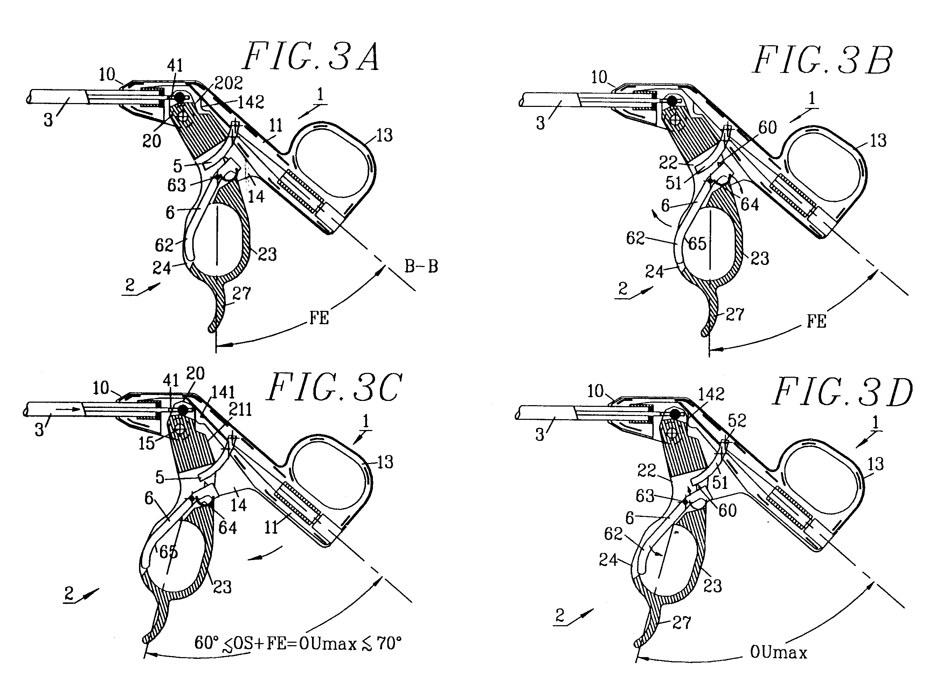

L'oscillation de la branche mobile 2 autour du pivot 15 a une amplitude OS dont les limites sont déterminées par l'angle formé entre deux portions de fond rectilignes 141 et 142 du logement 14. La première portion de fond 141 est située sensiblement à l'avant du corps de branche fixe 11 et forme avec l'axe BB de ce corps un petit angle aigu ayant un sommet au niveau du coude 12. La seconde portion de fond 142 est sensiblement perpendiculaire au tube 3 et située dans le coude 12. En position fermée de pince comme montré aux Figs. 1, 2A et 3A, suite à un rapprochement des anneaux de branche 13 et 23, un côté rectiligne 211 du corps de branche mobile 21 face au corps de branche fixe 11 bute contre la portion de fond 141 afin que la tête 20 avance la tige 4 en direction des mors MM et MF, la tête étant inclinée et dégagée de la seconde portion de fond 142. En position ouverte de pince, comme montré aux Figs. 2B et 3C, suite à un éloignement des anneaux 13 et 23, un côté latéral rectiligne 202 de la tête de branche mobile 20 situé au niveau du coude 12 bute contre la portion de fond 142 afin que la tête 20 recule la tige 4 vers le coude 12 ; le corps de branche mobile 21 est alors en majeure partie dégagé du logement 14, et la tête 20 est sensiblement verticale.

Sensiblement au centre de la branche mobile 2 est prévue une mortaise 22 traversant horizontalement le coude du corps 21 pour recevoir une portion crantée 51 de la crémaillère 5 saillant du corps de branche fixe 11, et un bras court 61 de la gâchette 6. La crémaillère 5 a un profil longitudinal en arc de cercle ayant pour centre le pivot 15 et a un chant convexe 50 muni de crans. Une seconde extrémité 52 de la crémaillère est fixée au corps 11. L'extrémité 52 est logée dans le fond d'un logement ménagé dans la demi-coque sus-jacente 1₂ plus profond que l'épaisseur de la crémaillère, ou dans un trou de la demi-coque 1₂.

La gâchette 6 est un barreau plat doublement coudé qui est monté à pivotement autour d'un pivot 63 fixé perpendiculairement aux faces de la branche mobile et traversant la mortaise 22. La gâchette 6 est partagée ainsi de part et d'autre du pivot 63 en un bras court 61 pouvant être déplacé dans la mortaise 22 et en un bras long 62 pouvant être déplacé dans une mortaise 24 qui traverse horizontalement un côté de l'anneau 23 orienté vers le tube 3 et qui communique avec la première mortaise 21. Le bras court 61 est muni d'un ergot 60 dont la pointe peut être engagée entre deux crans du chant de crémaillère 50. Dans le chant du bras de gâchette 61 opposée à l'ergot 60, sont encastrées les deux extrémités d'un ressort à lame demi-circulaire 64 s'appuyant contre un côté 25 de la mortaise 22 situé vers l'anneau 23.

Au repos, comme montré aux Figs. 1, 3A et 3D, le ressort à lame 64 pousse le court bras de gâchette 61 vers le chant 50 de la crémaillère afin que l'extrémité pointue de l'ergot 60 s'intercale entre deux crans de la crémaillère fixe 5. Dans ces conditions, la branche mobile 2 est immobilisée par rapport à la branche fixe, pour une portion donnée du mors mobile MM par rapport au mors fixe MF, et tout déplacement de la branche mobile est empêché. Au repos, le long bras de gâchette 62 offre un chant longitudinal courbe 65 qui saille de la seconde mortaise 24 et émerge dans le trou de l'anneau 23.

Pour désolidariser la gâchette 6 de la crémaillère 5, une poussée est exercée sur le chant de bras de gâchette 65 vers l'avant, en direction du tube de liaison 3, comme pour ouvrir une pince. Le bras 62 pénètre complètement dans la mortaise 24 et simultanément le bras court 61 de la gâchette 6 écrase sensiblement le ressort à lame 64 contre le côté de mortaise 25 afin de dégager l'ergot 60 des crans 50 de la crémaillère 51. La branche mobile 2 peut être alors rapprochée ou éloignée de la poignée fixe 1, par rotation autour du pivot 15, et corollairement, le mors mobile MM peut être rapproché ou éloigné du mors fixe MF afin de fermer ou ouvrir la pince.

Comme montré aux Figs. 2A et 2B, le mors fixe MF est solidaire d'une première extrémité 30 du tube de liaison 3, à l'avant de la pince.

Le mors fixe est, selon la réalisation illustrée, muni d'une suite de dents et d'un croc en regard de ceux du mors mobile MM afin de constituer une pince de dissection ou d'extraction. Toutefois, les mors peuvent présenter d'autres formes connues en fonction de l'usage de la pince. Selon une première variante, l'extrémité arrière creuse du mors fixe est soudée par laser au tube de liaison 3 quand ce dernier est en acier inoxydable. Selon une seconde variante, l'extrémité arrière creuse du mors fixe MF est emmanchée à force dans l'extrémité avant du tube de liaison 3 et collée à celle-ci lorsque le tube 3 est en matériau composite isolant.

Le mors mobile MM a une extrémité arrière pivotant autour d'un axe transversal AT fixé à l'arrière du mors fixe MF, et est relié à l'extrémité avant de la tige 4. Le mors mobile MM offre un profil, ici à dents et croc, analogue à celui du mors fixe MF.

Les deux mors sont métalliques, et sont typiquement en acier inoxydable ayant reçu une trempe suivie d'un revenu sous atmosphère neutre garantissant la longévité des mors.

Selon une autre variante, au lieu d'un seul mors mobile, les deux mors peuvent être mobiles sensiblement symétriquement par rapport à l'axe du tube, en pivotant autour d'un axe transversal au tube grâce à des liaisons des mors à l'extrémité avant de la tige 4. Une telle pince est particulèrement utilisée pour la dissection de tissus.

Le tube de liaison 3 a typiquement une longueur de 35 à 40 cm, et un diamètre interne de 2 mm. Selon la réalisation illustrée, le tube de liaison est issu d'un tube métallique en acier inoxydable, de diamètre externe 4,20 mm ; celui-ci est recouvert d'une gaine 33 en matière isolante électriquement, telle que TEFLON (marque déposée) thermorétractable ayant une épaisseur de 0,3 mm, afin de conférer un diamètre externe typiquement de 4,8 mm au tube de liaison, après rétreint de l'acier. De même, selon une seconde variante pour laquelle le tube 3 est en matériau composite isolant électriquement, le diamètre externe du tube est typiquement de 4,8 mm après passage à l'autoclave.

La tige 4 reliant l'arrière du mors mobile MM à la tête de branche mobile 20 est quelque peu plus longue que le tube de liaison 3 et a un diamètre, typiquement de 1,5 mm, sensiblement inférieur au diamètre interne du tube de liaison 3 pour glisser librement dans celui-ci. La tige 4 est en matériau conducteur électriquement, par exemple en acier inoxydable. Selon une autre variante, la tige peut être remplacée par un câble en acier.

En référence à nouveau à la Fig. 1, l'extrémité arrière 41 de la tige 4 sort de l'extrémité arrière 31 du tube de liaison 3 pour être fixée à la tête 20 de la branche mobile 2. L'extrémité arrière de tige 41 se termine dans la rainure de la chape de la tête 20 pour y être fixée par un axe de liaison transversal métallique 42, par exemple en acier inoxydable.

L'extrémité arrière 31 du tube de liaison 3 est emmanchée dans le nez 10 de la branche fixe 1 et a une partie non recouverte par la gaine 33 qui est soudée dans un fourreau 32 en matière conductrice électriquement, telle que laiton. Le fourreau 32 est encastré et collé dans un logement complémentaire ménagé axialement dans le nez 10 de la poignée fixe. Devant la base de l'extrémité arrière 31 du tube de liaison est prévu un joint cylindrique d'étanchéité 33 monté à frottement autour de la tige 4 et collé à l'intérieur du fourreau 32. Le joint 33 isole hermétiquement l'intérieur du tube 3 par rapport au logement 14 duquel émerge la tige 4 après avoir traversé le tube 3 et le fourreau 32, et donc isole l'environnement extérieur de l'extrémité avant du tube 3 et des mors MF et MM après pénétration dans le corps du patient.

A l'arrière du corps 11 de la branche fixe est prévue une prise de raccordement électrique monopolaire 7 pour relier les mors MM et MF à une borne positive d'une source de courant électrique SC, le corps du patient faisant office de terre. Une telle source de courant est utilisée notamment pour disséquer ou cautériser des tissus et vaisseaux.

La prise de raccordement 7 comporte un élément femelle conducteur constitué par un insert cylindrique 71 logé dans la branche fixe 1, et un élément conducteur mâle constitué par une fiche amovible 72 et enfichable dans l'insert 71 par l'arrière du corps 11, sous l'anneau 13. L'insert 71 et la fiche 72 sont en matériau conducteur électrique, tel que laiton, et disposés le long de l'axe BB du corps 11. L'insert 71 est encastré et collé dans un logement cylindrique complémentaire pratiqué dans le corps 11 et débouchant dans un lamage à l'arrière du corps. Le lamage reçoit en butée une extrémité avant d'un manchon de préhension 73 en matière isolante duquel saille la fiche 72. Le manchon est prolongé par une gaine isolante souple 74 contenant un fil électrique reliant la fiche 72 à la borne positive de la .source de courant SC. La gaine de fil électrique 74 tombe ainsi sous la main du chirurgien et ne gêne pas celui-ci au cours du geste opératoire.

Selon une autre variante, les éléments 71 et 72 peuvent être des éléments de prise mâle et femelle respectivement.

A partir de l'insert 71 et jusqu'au coude 12, au niveau de la portion de fond 142 du logement 14, s'étend un canal 16 ménagé longitudinalement dans le corps 11 de la branche fixe. En pratique, le canal 16 est prévu dans l'une 1₁ des demi-coques de la branche fixe 1 et passe sous l'extrémité fixée 52 de la crémaillère 5 logée dans l'autre demi-coque 1₂. Dans le fond du canal 16 est collé un fil électrique 17, par exemple en cuivre, ayant une première extrémité 171 soudée à l'insert 71 et une seconde extrémité 172 soudée au fourreau 32 du tube de liaison 3. Le fil 17 contourne la tête de branche mobile 20 dans le logement de branche fixe 14, et est plaqué contre le fond du logement 14 grâce à sa flexion propre lorsqu'il est courbé au niveau du coude 12, entre l'embouchure avant du canal 16 et le fourreau 32.

Le passage de courant électrique de l'insert de prise femelle 71 recevant le fiche 72 de la prise de raccordement 7 aux mors MF et MM est assuré à travers le fil électrique 17, le fourreau 32, et le tube de liaison 3 et accessoirement par contact fugitif de la tige 4 avec le tube 3. La branche fixe 1 étant en matière plastique et le tube 3 étant recouvert d'une gaine isolante 33, de résistivité diélectrique élevée, ceux-ci isolent parfaitement le chirurgien saisissant l'instrument. Bien que la gâchette 4 et la crémaillère 5 puissent être en matière conductrice électriquement, telle qu'acier inoxydable, il n'existe aucun risque que le chirurgien soit en contact électrique avec la source de courant, la demi-coque 1₂ plaquant, si nécessaire, le fil 17 dans le fond du canal 16 lors du recouvrement de l'autre demi-coque 1₁, et l'extrémité de crémaillère 52 complètement encastrée dans la demi-coque 1₂ ne pouvant être jamais au contact du canal 16 et donc du fil 17. L'isolation électrique ainsi réalisée ne peut être ainsi altérée au cours du temps et à l'usage.

Selon une seconde variante pour laquelle le tube 3 est en matériau composite isolant, la seconde extrémité 172 du fil 17 est soudée à l'extrémité 31 de la tige 31, en arrière de l'axe 42.

L'ergonomie des branches 1 et 2 est particulièrement conçue pour éviter toute fatigue de la main et assurer une prise naturelle de la pince. A cet effet, le corps de branche fixe 11 forme avec le tube de liaison 3 un angle obtu AO typiquement compris entre 130° et 140° environ. L'anneau fixe oblong 13 destiné à recevoir le pouce de la main a un axe longitudinal parallèle à l'axe de branche fixe BB d'une part, et au repos, mors MM et MF serrés, l'axe longitudinal de l'anneau oblong de branche mobile 23 destiné à recevoir le majeur de la main est sensiblement perpendiculaire au tube 3 et, en pratique, sensiblement verticale d'autre part. Ainsi les axes des deux anneaux forment un angle de fermeture FE compris entre 40° et 50° correspondant à une prise naturelle par la main. En outre, comparativement à un instrument à branches en acier, l'instrument selon l'invention est extrêmement plus léger.

La branche mobile 2 comporte, outre l'anneau 23 pour recevoir le majeur de la main du chirurgien, un coude dont le côté concave 26 longe la mortaise 22 orienté vers les mors devant la première extrémité 51 de la crémaillère 6, et une anse 27 qui prolonge longitudinalement l'anneau 23 et dont un côté orienté vers les mors est concave. Ainsi, le chant longitudinal de la branche mobile situé à l'opposé du premier anneau 13 présente un profil sensiblement sinusoïdal ayant une portion convexe centrale formée par un côté de l'anneau 23 et bordant la mortaise 24, une première portion concave formée par le côté 26 du coude pour recevoir l'index de la main, et une seconde portion concave formée par l'anse 27 pour recevoir l'annulaire de la main.

Par conséquent, la prise de la branche mobile est parfaitement confortable, même lorsque des poussées antagonistes sont exercées vers l'arrière par l'index et l'annulaire sur le côté 26 et l'anse 27, et vers l'avant par le majeur dans l'anneau 23 pour déplacer le long bras 62 de la gâchette 6.

S'agissant des moyens d'arrêt pour immobiliser la branche mobile 2 à une position souhaitée par rapport à la branche fixe 1, et donc pour sélectionner une ouverture entre les mors MF et MM, la crémaillère 5 est complètement dissimulée dans le corps de branche fixe 11 et dans le logement de branche fixe 14 et la mortaise de branche mobile 22, ce qui évite toute blessure des doigts par les crans de la crémaillère et coincement de ceux-ci avec la crémaillère. En outre, bien que la crémaillère 5 ne soit pas directement accessible, celle-ci est néanmoins isolée électriquement du fil conducteur 17.

La crémaillère 5 est ainsi constamment immobile et n'est aucunement sollicitée en flexion pour arrêter la branche mobile 2, comme cela apparait ci-dessous en référence aux Figs 3A à 3D.

Il est supposé à la Fig. 3A que les branches ont été rapprochées afin de serrer les mors MF et MM pour introduction dans le corps du patient. Le côté de corps de branche mobile 211 est maintenu en butée contre la première portion de fond de logement 141 dans la branche fixe 1, et l'ergot 60 de la gâchette 6 est ancré dans la crémaillère 5, à proximité du bras 11. L'angle de fermeture FE entre les branches est typiquement compris entre 40° et 50°.

Pour desserrer les mors MM et MF, le majeur de la main introduit dans l'anneau de branche mobile 23 tend naturellement à écarter la branche mobile de la branche fixe. Cette sollicitation naturelle se traduit par une poussée sur le long bras de gâchette 62 saillant à l'intérieur de l'anneau 23, développée par la première phalange du majeur. L'extrémité du long bras de gâchette dont la largeur et le profil sont sensiblement identiques à ceux de l'anneau 23 au niveau de la mortaise 24, pénêtre complètement dans cette mortaise lorsque la phalange du majeur bute contre l'embouchure de la mortaise dans l'anneau.

La gâchette 6 tourne ainsi autour du pivot 63 suivant le sens des aiguilles d'une montre, comme montré à la Fig. 3B, afin de dégager l'ergot de gâchette 60 à l'encontre de la poussée du ressort à lame 64 s'écrasant sensiblement.

La poussée du majeur de la main exercée dans l'anneau 23 est poursuivie jusqu'à l'ouverture souhaitée entre les mors MM et MF, la tige 4 glissant vers l'arrière dans le tube 3. L'angle d'ouverture OU maximum entre les branches 1 et 2 lorsque le bord de tête 202 est en butée contre la portion de fond de logement 142, est typiquement compris entre 60 et 70°, pour une amplitude d'oscillation OS de la branche mobile autour du pivot 15 de 20° environ, comme montrée à la fig. 3C.

A l'ouverture souhaitée, la pression exercée par le majeur de la main cesse. Le ressort à lame 64 pousse le court bras de gâchette 61 vers la crémaillère 5 afin que la pointe de l'ergot de gâchette 60 s'insère entre deux crans de la crémaillère et donc arrête la poignée mobile à la position souhaitée. Simultanément, comme montré à la Fig. 3D, la rotation de la gâchette 6 suivant le sens contraire à celui des aiguilles d'une montre autour du pivot 63 conduit le chant concave 65 du long bras de gâchette 62 à saillir dans l'anneau 23.

Le chirurgien procède de la même manière que ci-dessous chaque fois qu'il souhaite arrêter la poignée mobile à une position prédéterminée correspondant à un écartement déterminé des mors MM et MF.

Il apparaît que le passage d'une position de la branche mobile 2 à une autre position est naturel, comme pour une paire de ciseaux, et la rotation de la branche 2 autour du pivot 15 est libre et n'est point freinée par un quelconque frottement de l'ergot de gâchette 60 contre les crans 50 de la crémaillère 5.

## Revendications

1. Instrument chirurgical comprenant :
- une première branche (1) ayant un coude (12) le partageant en un nez (10) et un corps (11) dont l'extrémité opposée au coude comporte un premier anneau de préhension (13),
- une seconde branche (2) articulée autour d'un pivot (15) fixé sensiblement à l'intérieur du coude (12) de la première branche, la seconde branche comportant de part et d'autre du pivot, une tête (20) et un corps dont l'extrémité opposée au pivot comporte un second anneau de préhension (23),
- un tube de liaison (3) ayant une première extrémité (30) supportant deux mors (MF, MM) dont l'un (MM) au moins est mobile, et une seconde extrémité (31) fixée au nez (10),
- une tige (4) traversant le tube de liaison (3) et reliant le mors mobile (MM) à la tête de seconde branche (20),
- une crémaillère (5) ayant une première extrémité (51) coopérant avec un moyen d'arrêt (6) relié au corps de seconde branche (21), et une seconde extrémité (52) fixée au corps de première branche (11), et
- des moyens de liaison électrique (17, 7) solidaires de la première branche pour relier électriquement les mors (MF, MM) à des moyens d'alimentation électrique monopolaire (SC),
caractérisé en ce que les première et seconde branches (1,2) sont en matière isolante électriquement pour isoler la crémaillère (5) et le moyen d'arrêt (6) par rapport aux moyens de liaison électrique (17,7), et la crémaillère (5) et le moyen d'arrêt (6) sont logés à l'intérieur des corps de branche (11, 21).

2. Instrument conforme à la revendication 1, caractérisé en ce que la première branche (1) est constituée de deux demi-coques (1₁, 1₂) sensiblement plates et moulées,et la seconde branche (2) est monolithique et moulée.

3. Instrument conforme à la revendication 1 ou 2, caractérisé en ce que la première branche (1) comporte un logement ouvert (14) ménagé à l'intérieur du coude (12) et du corps de première branche (11) pour loger la tête (20) et une partie du corps de seconde branche (21), et ladite partie du corps de seconde branche (21) comporte une mortaise (22) recevant la seconde extrémité (51) de la crémaillère.

4. Instrument conforme à la revendication 3, caractérisé en ce que l'amplitude d'oscillation de la seconde branche (2) autour du pivot (15) est limitée par une première portion de fond (141) du logement (14) dans le corps de première branche (11) faisant office de butée pour un côté (211) du corps de seconde branche (21), et par une seconde portion de fond (142) du logement (14) faisant office de butée pour un côté (202) de la tête de seconde branche (20), ladite amplitude étant de préférence de 20° environ.

5. Instrument conforme à l'une quelconque des revendications 1 à 4, caractérisé en ce que
le moyen d'arrêt est une gâchette (6) pivotante autour d'un second pivot (63) fixé dans le corps (21) de la seconde branche,
ladite gâchette est partagée par le second pivot en un premier bras (61) logé dans une première mortaise (22) ménagée dans le corps de seconde branche (21) et recevant la seconde extrémité (51) de la crémaillère (5), et un second bras (62) logé dans une seconde mortaise (24) ménagée dans le second anneau (23),
le premier bras (61) est engagé avec des crans (50) de la seconde extrémité de crémaillère (51) et le second bras (62) est partiellement saillant à l'intérieur du second anneau (23) sous l'action d'un moyen de ressort (64), et
le premier bras (61) est dégagé des crans de crémaillère (50) et le second bras (62) est contenu complètement dans la seconde mortaise (24) sous l'action d'une poussée exercée de l'intérieur du second anneau (23) sur le second bras et à l'encontre du moyen de ressort (64).

6. Instrument conforme à la revendication 5, caractérisé en ce que le moyen de ressort est un ressort à lame (64) fixé au premier bras (61) et appliqué contre un côté (25) de la première mortaise (22).

7. Instrument conforme à la revendication 5 ou 6, caractérisé en ce que le second bras de gâchette (62) a un profil épousant sensiblement celui du second anneau (23).

8. Instrument conforme à l'une quelconque des revendications 5 à 7, caractérisé en ce que la seconde mortaise (24) est située dans un côté du second anneau (23) orienté vers les mors (MF, MM) et à l'opposé du premier anneau (13).

9. Instrument conforme à l'une quelconque des revendications 1 à 8, caractérisé en ce que la seconde branche (2) présente un chant sensiblement sinusoïdal orienté vers les mors (MF, MM) et à l'opposé du premier anneau (13), ledit chant comportant une portion convexe centrale formée par un côté (24) du second anneau (23), une première portion concave (26) située devant la première extrémité de crémaillère (51), et une seconde portion concave formée par un côté d'une anse (27) liée au second anneau.

10. Instrument conforme aux revendications 5 et 9, caractérisé en ce que ladite portion convexe centrale du chant sinusoïdal borde la seconde mortaise (24).

11. Instrument conforme à l'une quelconque des revendications 1 à 10, caractérisé en ce que les moyens de liaison électrique comportent un premier élément conducteur (71) logé dans l'arrière du corps de première branche (11) au niveau du premier anneau (13) pour être assemblé avec un second élément conducteur (72) de prise de raccordement électrique monopolaire (7) relié aux moyens d'alimentation (SC), et un fil électrique (17) cheminant dans un canal (16) qui s'étend longitudinalement dans la première branche et isole le fil de la crémaillère (5), ledit fil ayant une première extrémité (171) liée au premier élément conducteur (71) et une seconde extrémité (172) située dans le nez et liée au tube de liaison (3).

12. Instrument conforme à la revendication 11, caractérisé en ce que le tube (3) est en matériau conducteur recouvert par une gaine isolante (33).

13. Instrument conforme à la revendication 11, caractérisé en ce que la liaison de la seconde extrémité de fil (172) au tube de liaison (3) est remplacée par une liaison de la seconde extrémité de fil (172) à la tige (4), le tube de liaison (3) étant en matière isolante électriquement.

14. Instrument conforme à l'une quelconque des revendications 1 à 13, caractérisé en ce que la seconde extrémité de tube (31) est emmanchée dans un fourreau (32) logé dans le nez de première branche (10) et est prolongée dans le fourreau par un joint (33) traversé à frottement par la tige (4) pour étanchéifier l'intérieur du tube par rapport à l'environnement extérieur.

15. Instrument conforme à l'une quelconque des revendications 1 à 14, caractérisé en ce que l'angle de coude (AO) délimité par le nez (10) et le corps de première branche (11) et contenant la seconde branche (2) est compris entre 130° et 140° environ.

## Claims

1. A surgical instrument comprising:
- a first leg (1) having a bend (12) dividing it into a nose (10) and a body (11), the end of the body remote from the bend comprising a first gripping ring (13),
- a second leg (2) articulated around a pivot (15) secured substantially inside the bend (12) of the first leg, the second leg comprising a head (20) on one side of the pivot and a body on the other side, the end of the body remote from the pivot comprising a second gripping ring (23),
- a connecting tube (3) having a first end (30) bearing two jaws (MF, MM) at least one (MM) of which is movable, and a second end (31) fixed to the nose (10),
- a rod (4) extending through the connecting tube (3) and connecting the movable jaw (MM) to the head of the second leg (20),
- a rack (5) having a first end (51) co-operating with a stop means (6) connected to the body (21) of the second leg, and a second end (52) secured to the body (11) of the first leg, and
- electric connection means (17, 7) secured to the first leg and electrically connecting the jaws (MF, MM) to unipolar electric supply means (SC),
characterised in that the first and second legs (1, 2) are of electrically insulating material for insulating the rack (5) and the stop means (6) from the electric connection means (17, 7), and the rack (5) and the stop means (6) are disposed inside the leg bodies (11, 21).

2. An instrument according to claim 1, characterised in that the first leg (1) comprises two substantially flat, moulded half-shells (1₁, 1₂), and the second leg (2) is monolithic and moulded.

3. An instrument according to claim 1 or 2, characterised in that the first leg (1) has an open compartment (14) formed inside the bend (12) and the body (11) of the first leg for holding the head (20) and a part of the body (21) of the second leg, and the aforementioned part of the body (21) comprises a slot (22) receiving the second end (51) of the rack.

4. An instrument according to claim 3, characterised in that the amplitude of oscillation of the second leg (2) around the pivot (15) is limited by a first bottom portion (141) of the compartment (14) in the body (11) of the first leg and serving as an abutment for a side (211) of the body (21) of the second leg, and by a second bottom portion (142) of the compartment (14) serving as an abutment for one side (202) of the head (20) of the second leg, the amplitude being preferably about 20°.

5. An instrument according to any of claims 1 to 4, characterised in that
the stop means is a pawl (6) pivoting around a second pivot (63) secured in the body (21) of the second leg,
the pawl is divided by the second pivot into a first arm (61) disposed in a first slot (22) formed in the body (21) of the second leg and receiving the second end (51) of the rack (5), and a second arm (62) disposed in a second slot (24) formed in the second ring (23),
the first arm (61) engages in notches (50) in the second end (51) of the rack, and the second arm (62) partially projects into the second ring (23) under the action of a spring means (64), and
the first arm (61) is disengaged from the notches (50) in the rack and the second arm (62) is completely contained in the second slot (24) as a result of a thrust exerted from the interior of the second ring (23) on the second arm and against the spring means (64).

6. An instrument according to claim 5, characterised in that the spring means is a leaf spring (64) secured to the first arm (61) and pressed against one side (25) of the first slot (22).

7. An instrument according to claim 5 or 6, characterised in that the second arm (62) of the pawl has a cross-section which substantially matches that of the second ring (23).

8. An instrument according to any of claims 5 to 7, characterised in that the second slot (24) is situated on one side of the second ring (23) facing towards the jaws (MF, MM) and remote from the first ring (13).

9. An instrument according to any of claims 1 to 8, characterised in that the second leg (2) has a substantially sinusoidal end directed towards the jaws (MF, MM) and remote from the first ring (13), the edge comprising a central convex portion formed by one side (24) of the second ring (23), a first concave portion (26) situated in front of the first end (51) of the rack, and a second concave portion formed by one side of a loop (27) connected to the second ring.

10. An instrument according to claims 5 and 9, characterised in that the central convex portion of the sinusoidal edge is adjacent the second slot (24).

11. An instrument according to any of claims 1 to 10, characterised in that the electric connecting means comprise a first conductive element (71) disposed in the rear of the body (11) of the first leg at the level of the first ring (13) so as to be fitted to a second conductive element (72) of a unipolar electric power connection (7) connected to the supply means (SC), and also comprise an electric wire (17) disposed in a duct (16) which extends longitudinally in the first leg and insulates the wire from the rack (5), the wire having a first end (171) connected to the first conductive element (71) and a second end (172) situated in the nose and connected to the connecting tube (3).

12. An instrument according to claim 11, characterised in that the tube (3) is of conductive material covered by an insulating sheath (33).

13. An instrument according to claim 11, characterised in that the connection between the connecting tube (3) and the second end (172) of the wire is replaced by a connection between the rod (4) and the second end (172) of the wire, the connecting tube (3) being made of electrically insulating material.

14. An instrument according to any of claims 1 to 13, characterised in that the second end (31) of the tube is fitted into a sheath (32) disposed in the nose (10) of the first leg and is prolonged in the sheath by a seal (33) through which the rod (4) extends with friction, thus sealing the interior of the tube from the external environment.

15. An instrument according to any of claims 1 to 14, characterised in that the angle of the bend (AO) defined by the nose (10) and the body (11) of the first leg and containing the second leg (2) is between about 130° and 140°.

## Patentansprüche

1. Chirurgisches Instrument, umfassend:
- einen ersten Griffabschnitt (1) mit einem Winkelstück (12), der ihn in ein Vorderteil (10) und ein Hauptstück (11) aufteilt, dessen dem Winkelstück entgegengesetztes Ende einen ersten Greifring (13) umfaßt,
- einen zweiten Griffabschnitt (2), der um eine Schwenkachse (15) gelenkig angebracht ist, die im wesentlichen im Inneren des Winkelstücks (12) des ersten Griffabschnitts befestigt ist, wobei der zweite Griffabschnitt auf beiden Seiten der Schwenkachse einen Kopf (20) und ein Hauptstück umfaßt, dessen der Schwenkachse entgegengesetztes Ende einen zweiten Greifring (23) umfaßt,
- ein Verbindungsrohr (3) mit einem ersten Ende (30), das zwei Backen (MF, MM) trägt, von denen wenigstens eine (MM) beweglich ist, und mit einem an dem Vorderteil (10) befestigten zweiten Ende (31),
- eine Stange (4), die das Verbindungsrohr (3) durchquert und die bewegliche Backe (MM) mit dem Kopf des zweiten Griffabschnitts (20) verbindet,
- eine Zahnstange (5) mit einem ersten Ende (51), das mit einem mit dem Hauptstück des zweiten Griffabschnitts (21) verbundenen Stopp-Mittel (6) zusammenwirkt, und mit einem zweiten Ende (52), das an dem Hauptstück des ersten Griffabschnitts (11) befestigt ist, und
- elektrische Verbindungsmittel (17, 7), die mit dem ersten Griffabschnitt verbunden sind, um die Backen (MF, MM) mit Mitteln zur elektrischen einpoligen Versorgung (SC) elektrisch zu verbinden,
dadurch **gekennzeichnet,** daß die ersten und zweiten Griffabschnitte (1, 2) aus elektrisch isolierendem Material sind, um die Zahnstange (5) und das Stopp-Mittel (6) in bezug auf die elektrischen Verbindungsmittel (17, 7) zu isolieren, und die Zahnstange (5) und die Stopp-Mittel (6) im Inneren der Griffabschnitthauptkörper (11, 21) untergebracht sind.

2. Instrument nach Anspruch 1, dadurch **gekennzeichnet,** daß der erste Griffabschnitt (1) aus zwei im wesentlichen flachen und geformten Halbschalen (1₁,1₂) besteht und der zweite Griffabschnitt (2) monolithisch und geformt ist.

3. Instrument nach Anspruch 1 oder 2, dadurch **gekennzeichnet,** daß der erste Griffabschnitt (1) eine im Inneren des Winkelstücks (12) und des Hauptstücks des ersten Griffabschnitts (11) ausgesparte offene Aufnahme (14) umfaßt, um den Kopf (20) und einen Teil des Hauptstücks des zweiten Griffabschnitts (21) unterzubringen, und der Teil des Hauptstücks des zweiten Griffabschnitts (21) eine Aussparung (22) umfaßt, die das zweite Ende (51) der Zahnstange aufnimmt.

4. Instrument nach Anspruch 3, dadurch **gekennzeichnet,** daß die Hin- und Herbewegungsamplitude des zweiten Griffabschnitts (2) um die Schwenkachse (15) durch einen ersten Bodenabschnitt (141) der Aufnahme (14) im Hauptstück des ersten Griffabschnitts (11), der als Anschlag für eine Seite (211) des Hauptstücks des zweiten Griffabschnitts (21) dient, und durch einen zweiten Bodenabschnitt (142) der Aufnahme (14) begrenzt ist, der als Anschlag für eine Seite (202) des Kopfes des zweiten Griffabschnitts (20) dient, wobei die Amplitude vorzugsweise etwa 20° ist.

5. Instrument nach einem beliebigen der Ansprüche 1 bis 4, dadurch **gekennzeichnet,** daß
- das Stopp-Mittel ein Drücker (6) ist, der um eine zweite Schwenkachse (63) schwenkbar ist, die im Hauptstück (21) des zweiten Griffabschnitts befestigt ist,
- der Drücker durch die zweite Schwenkachse und einen ersten Arm (61), der in einer ersten Aussparung (22), die im Hauptstück des zweiten Griffabschnitts (21) ausgespart ist und das zweite Ende (51) der Zahnstange aufnimmt, und einen zweiten Arm (62) aufgeteilt ist, der in einer zweiten Aussparung (24) untergebracht ist, die im zweiten Ring (23) ausgespart ist,
- sich der erste Arm (61) unter der Wirkung eines Federmittels (64) in Eingriff mit Kerbungen (50) des zweiten Endes der Zahnstange (51) befindet und der zweite Arm (62) teilweise in das Innere des zweiten Rings (23) vorsteht, und
- unter der Wirkung eines Drucks, der vom Inneren des zweiten Rings (23) auf den zweiten Arm und gegen das Federmittel (64) ausgeübt wird, der erste Arm (61) von den Zahnstangenkerbungen (50) außer Eingriff gebracht ist und der zweite Arm (62) vollständig in der zweiten Aussparung (24) enthalten ist.

6. Instrument nach Anspruch 5, dadurch **gekennzeichnet,** daß das Federmittel eine Blattfeder (64) ist, die am ersten Arm (61) befestigt ist und gegen eine Seite (25) der ersten Aussparung (22) angesetzt ist.

7. Instrument nach Anspruch 5 oder 6, dadurch **gekennzeichnet,** daß der zweite Drückerarm (62) ein Profil hat, das sich im wesentlichen an dasjenige des zweiten Rings (23) anschließt.

8. Instrument nach einem beliebigen der Ansprüche 5 bis 7, dadurch **gekennzeichnet,** daß die zweite Aussparung (24) in einer Seite des zweiten Rings (23) liegt, die zu den Backen (MF, MM) und entgegengesetzt zum ersten Ring (13) orientiert ist.

9. Instrument nach einem beliebigen der Ansprüche 1 bis 8, dadurch **gekennzeichnet,** daß der zweite Arm (2) eine Kante im wesentlichen sinusförmig orientiert zu den Backen (MF, MM) und entgegengesetzt zum ersten Ring (13) aufweist, wobei die Kante einen durch eine Seite (24) des zweiten Rings (23) gebildeten zentralen konvexen Abschnitt, einen vor dem ersten Zahnstangenende (51) liegenden konkaven Abschnitt (26) und einen durch eine Seite eines mit dem zweiten Ring verbundenen Bügels (27) gebildeten konkaven zweiten Abschnitt umfaßt.

10. Instrument nach den Ansprüchen 5 und 9, dadurch **gekennzeichnet,** daß der zentrale konvexe Abschnitt der sinusförmigen Kante die zweite Aussparung (24) begrenzt.

11. Instrument nach einem beliebigen der Ansprüche 1 bis 10, dadurch **gekennzeichnet,** daß die Mittel zur elektrischen Verbindung ein erstes leitendes Element (71), das am hinteren Teil des Hauptstücks des ersten Griffabschnitts (11) in der Höhe des ersten Rings (13) zur Verbindung mit einem zweiten leitenden Element (72) für einen einpoligen elektrischen Anschluß (7) untergebracht ist, das mit den Versorgungsmitteln (SC) verbunden ist, und einen elektrischen Leiter (17) umfassen, der durch einen Kanal (16) geht, der sich in Längsrichtung im ersten Griffabschnitt erstreckt und den Leiter gegen die Zahnstange (5) isoliert, wobei der Leiter ein mit dem ersten leitenden Element (71) verbundenes erstes Ende (171) und ein zweites Ende (172) aufweist, das sich im Vorderteil befindet und mit dem Verbindungsrohr (3) verbunden ist.

12. Instrument nach Anspruch 11, dadurch **gekennzeichnet,** daß das Rohr (3) aus leitendem Material ist, überdeckt von einer isolierenden Ummantelung (33).

13. Instrument nach Anspruch 11, dadurch **gekennzeichnet,** daß die Verbindung des zweiten Leiterendes (172) mit dem Verbindungsrohr (3) durch eine Verbindung des zweiten Leiterendes (172) mit der Stange (4) ersetzt ist, wobei das Verbindungsrohr (3) aus elektrisch isolierendem Material ist.

14. Instrument nach einem beliebigen der Ansprüche 1 bis 13, dadurch **gekennzeichnet,** daß das zweite Rohrende (31) in einer Hülse (32) eingesetzt ist, die im Vorderteil des ersten Griffabschnitts (10) untergebracht ist und in der Hülse durch eine Dichtung (33) mit Reibung durch die Stange (4) durchquert ist, um das Innere des Rohrs in bezug auf die Außenumgebung abzudichten.

15. Instrument nach einem beliebigen der Ansprüche 1 bis 14, dadurch gekennzeichnet, daß der Winkel des Winkelstücks (AO), der durch das Vorderteil (10) und das Hauptstück des ersten Griffabschnitts (11) begrenzt ist und den zweiten Griffabschnitt (2) enthält, enthalten ist zwischen etwa 130° und 140°.
